# EUROPÄISCHE PATENTSCHRIFT

(11) **EP 0 628 295 B1**
(45) Veröffentlichungstag und Bekanntmachung des Hinweises auf die Patenterteilung: **03.11.1999**
(21) Anmeldenummer: 94250124.8
(22) Anmeldetag: 11.05.1994
(51) Int. Cl.: A61F 2/46

(54) **Einfüllvorrichtung für Knochenzement**
Filling apparatus for bone cement
Dispositif de remplissage pour ciment à os

(30) Priorität: 11.05.1993 DE 4316655
(43) Veröffentlichungstag der Anmeldung: 14.12.1994
(73) Patentinhaber: ARTOS Medizinische Produkte GmbH, 12277 Berlin (DE)
(72) Erfinder: Bernoski, Franziskus Pieter, Dr., NL-2597 AD Den Haag (NL); Kranz, Curt, Dr.-Ing., D-10825 Berlin (DE)
(74) Vertreter: Eisenführ, Speiser & Partner

(56) Entgegenhaltungen:
- EP-A- 0 093 560
- EP-A- 0 428 127
- WO-A-90/04953
- DE-U- 8 624 398
- GB-A- 2 052 267
- GB-A- 2 105 198
- US-A- 4 625 722

## Beschreibung

Die Erfindung betrifft eine Einfüllvorrichtung der im Oberbegriff des Anspruchs 1 angegebenen Art.

Aus der EP-A2 0 093 560 ist eine Einfüllvorrichtung für Knochenzement in den intramedullären Kanal eines Knochens bekannt. Die Vorrichtung weist eine Kanüle auf, durch die der Knochenzement in den Kanalhohlraum eingebracht werden kann. Zur Stabilisierung der Kanülenposition innerhalb des intramedullären Kanals ist eine aufblasbare Manschette vorgesehen, die den Schaft der Kanüle allseitig umschließt Diese Manschette verhindert gleichzeitig, daß beim Verpressen des Knochenzements am Boden des intramedullären Knochenkanals überschüssiges Material in den an der Kanülenaustrittsöffnung befindlichen Ringraum zwischen der Außenwandung der Kanüle und der Innenwandung des Knochenkanals eindringen kann. Diese ungewünschte Materialbewegung muß insbesondere deshalb unterbunden werden, weil das Eindringen des Knochenzements in den Ringraum oberhalb des Kanülenausganges eine Reduzierung des verfülldruckes zur Folge hätte.

Die vorstehend beschriebene Lösung weist jedoch den Nachteil auf, daß die Einfüllvorrichtung an ihrem proximalen Ende durch die aufblasbare Dichtung in radialer Richtung erhebliche Abmessungen aufweist. Es ist somit erforderlich, den intramedullären Knochenkanal über das an sich erforderliche Maß hinaus radial zu vergrößern, womit ein zusätzlicher Verlust von gesundem Knochenmaterial verbunden ist. Darüberhinaus ist für die Benutzung der Einfüllvorrichtung Hilfsenergie erforderlich, um die Dichtmanschette mit dem erforderlichen Innendruck zu versehen.

Weil die bekannte Dichtmanschette auf der Einfüllkanüle verschieblich angeordnet ist, ist die Handhabbarkeit der Einfüllvorrichtung zusätzlich dadurch erschwert, daß die Manschette nicht ohne besonders Übung an den Verfüllpunkt am Ende des intramedullären Knochenkanals positioniert werden kann. Dies ist besonders deshalb ungünstig, da die Höhe der Verfüllung mit Knochenzement nicht exakt festgelegt werden kann. Weiterhin ist nachteilig, daß die Dichtmanschette nach Beendigung der verfüllung wieder entlüftet werden muß.

Aus GB-A-2 105 198 und US-A-4 625 722 ist jeweils eine Einfüllvorrichtung für Knochenzement gemäß dem Oberbegriff von Anspruch 1 bekannt.

Hierbei ist am freien Ende einer Einfüllkanüle eine im Ausgangszustand zusammengefaltete und im Zuge des Ausbringens des Knochenzements radial aufweitbare Manschette als Leitmittel für den Zement angeordnet.

Die Herstellung der mit dieser Manschette versehenen Einfüllkanüle ist relativ aufwendig, und bei einigen Ausführungsformen dürfte auch die Handhabung einige Übung erfordern.

Ausgehend von den Mängeln des Standes der Technik, liegt der Erfindung die Aufgabe zugrunde, eine Einfüllvorrichtung der eingangs genannten Gattung zu entwickeln, mit der mit einfachen Mitteln eine Verfüllung des Knochenkanals vorgenommen werden kann, ohne daß der eingefüllte Zement an der Kanüle aufsteigt.

Diese Aufgabe wird mit den Merkmalen des Anspruchs 1 gelöst. der Erfindung weist eine flexible Fortsetzung der Einfüllkanüle eine zu ihrem freien Ende hin zunehmende, durch Überlappung, gebildet durch eine lamellenartige Schlitzung des Leitmittels in Austrittsrichtung des Knochenzements, oder Elastizität gebildete Materialreserve auf, die trotz Aufspreizens des freien Endes durch den austretenden Knochenzement eine geschlossene Manschette bildet, welche ein Aufsteigen des Knochenzements an der Kanüle verhindert. Durch den sich im Innern der Manschette aufbauenden Druck des Knochenzements legt sich diese dichtend an die Innenwand des Knochenkanals an und hält auf diese Weise einen Überdruck aufrecht, welche den Knochenzement in die Trabekelstruktur preßt, so daß eine innige Verbindung von Zement und Knochenwandung erreicht wird. Insbesondere ist auch das Entstehen von Lufteinschlüssen ausgeschlossen.

Entsprechend einer bevorzugten Ausführungsform der Erfindung ist das freie Ende der Einfüllkanüle faltenrockähnlich erweitert, wobei gleichzeitig eine Verringerung der Wandstärke in Richtung des Materialaustrags vorgesehen ist. Während des Verfüllens des intramedullären Knochenkanals wird das proximale Ende der Einfüllkanüle durch die Druckwirkung des Verfüllmaterials im wesentlichen in radialer Richtung gedehnt, bis ein Wirkkontakt mit der Innenwand des Knochenkanals eingetreten ist. Das freie Ende der Einfüllkanüle weist danach insbesondere die Form eines Kegelstumpfes auf. Durch eine ausreichende Welligkeit der proximalen Abschlußkante der Einfüllkanüle bei gleichzeitig großer Elastizität des verwendeten Materials kann sich der Endbereich der Einfüllkanüle - durch die so gebildete Materialreserve - der Querschnittsveränderung des intramedullären Knochenkanals anpassen und eine ausreichende Abdichtung gewährleisten, wenn die Einfüllkanüle während des Einfüllvorgangs in dem Knochenkanal allmählich nach oben bewegt wird.

Die erfindungsgemäßen Leitmittel werden bevorzugt einstückig an das Ende der Einfüllkanüle angeformt. Für den Fall, daß der Werkstoff, aus dem die Einfüllkanule gefertigt ist, unter Einsatzbedingungen nur eine geringe Elastizität aufweist, ist entsprechend einer anderen vorteilhaften Weiterbildung der Erfindung an dem freien Ende der Einfüllkanüle die expandierbare Manschette alz zusätzliches Element vorgesehen. Diese kann in Form eines elastischen ringförmigen Elements aufgeklemmt, geklebt oder in sonstiger Weise dichtend verbunden sein.

Die Manschette besteht vorzugsweise aus Gummi oder einem entsprechenden elastischen Werkstoff und ist am proximalen Ende der Außenfläche der Einfüllkanüle mit dieser durch Kleben verbunden. Diese Verbindungstechnik führt in günstiger Weise zu keiner nennenswerten Vergrößerung des Außendurchmessers der Einfüllkanüle.

Entsprechend einer anderen günstigen Weiterbildung der Erfindung läuft das proximale Ende der Einfüllkanüle in eine Mehrzahl von Streifenelementen aus. Die Streifenelemente sind im wesentlichen gleichartig ausgebildet und gleichmäßig auf den Umfang der Einfüllkanüle verteilt angeordnet. Sie bilden am freien Ende der Einfüllkanüle einen zylindrischen Rohrstutzen, der in radialer Richtung verformt werden kann. Das Maß der Verformung nimmt dabei in Austrittsrichtung des Verfüllmaterials zu, so daß das proximale Ende der Einfüllkanüle unter Betriebsbedingungen im wesentlichen in eine kegelstumpfartige Konfiguration übergeht. Dabei werden die Außenseiten der Streifenelemente durch das Verfüllmaterial gegen die In-nenwandung des intramedullären Knochenkanals gepreßt und dichten das proximale Ende der Einfüllkanüle in gewünschter Weise gegenüber der Innenwandung des intramodularen Knochenkanals ab.

Die am proximalen Ende der Einfüllkanüle befindlichen Streifenelemente sind gegebenenfalls einlagig angeordnet, wobei die Kanten der Schmalseiten der Streifenelemente am Umfang der Einfüllkanüle im wesentlichen tangential verlaufend angeordnet sind. Die Längskanten der Streifenelemente überlappen ein-ander jeweils soweit, daß bei der unter Einsatzbedingungen maximal zu erwartenden radialen Verformung eine geschlossene Wandung erhalten bleibt, um das Austreten des Verfüllmaterials in eine unerwünschte Richtung zu verhindern. Der Querschnitt der Anordnung ist dann sägezahnartig.

Entsprechend einer weiteren günstigen Ausführungsform der Erfindung sind die am proximalen Ende der Einfüllkanüle befindlichen Streifenelemente doppellagig angeordnet. Die Streifenelemente der oberen Lage sind derart angeordnet, daß sie die zwischen den Streifenelementen der unteren Lage befindlichen Spalten überdecken und beim Verpressen des Füllmaterials eine zuverlässige Dichtung gewährleisten, so daß das Verfüllmaterial nur in der vorgesehenen Richtung in den Knochenkanal austreten kann. Bei dieser Anordnung der Streifenelemente ist es in fertigungstechnischer Hinsicht günstig, die erste (untere) Lage von Streifenelementen direkt an das freie Ende der Einfüllkanüle anzuformen und die zweite Streifenelementenlage in Form einer Manschette auf das frei Ende der Einfüllkanüle aufzuschieben und in geeigneter Weise, insbesondere durch Kleben, zu befestigen.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 ein vorteilhaftes Ausführungsbeispiel der Erfindung bei Benutzung im Längsschnitt,
Figur 2 ein Detail einer ersten Variante der Erfindung in vergrößerter perspektivischer Darstellung,
Figur 3 das Detail gemäß Figur 2 in der Draufsicht,
Figur 4 eine andere vorteilhafte Ausführungsform des Details gemäß Figur 3,
Figur 5 das Detail gemäß Figur 4 in der Draufsicht,
Figur 6 eine andere vorteilhafte Ausführungsform des Details gemäß Figur 2, sowie
Figur 7 das Detail gemäß Figur 6 in der Draufsicht.

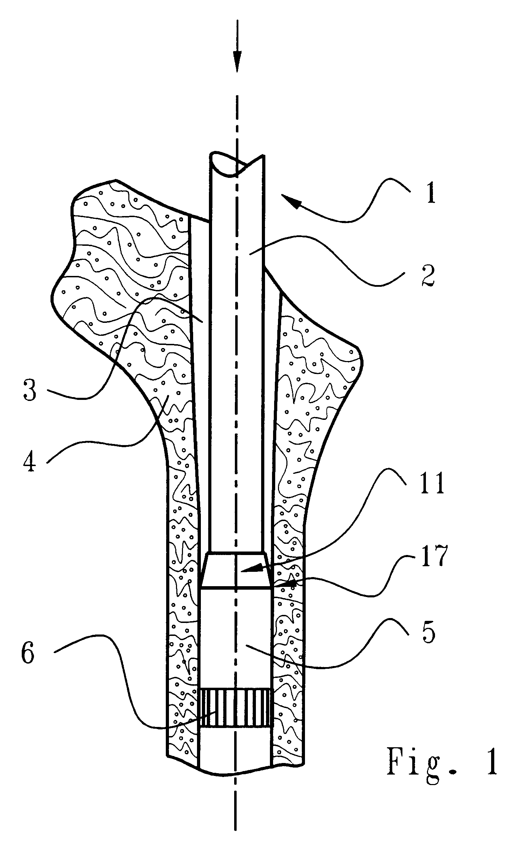

Figur 1 zeigt in schematisierter Darstellung einen Längsschnitt durch einen Knochen 4, der einen im wesentlichen in Richtung seiner Längsachse verlaufenden intramedullären Knochenkanal 3 aufweist. In diesen Kanal 3 ist die Einfüllkanüle 2 einer Einfüllvorrichtung 1 eingebracht. Das freie Ende 11 der Einfüllkanüle 2 ist in radialer Richtung elastisch verformbar und wird durch das aus der Einfüllkanüle 2 austretende Verfüllmaterial 5 gegen die innere Wandung des intramedullären Knochenkanals 3 gepreßt. Dadurch entsteht eine im wesentlichen ringförmige Dichtung 17, die sich stets in der Übergangszone vom verfüllten zum unverfüllten Abschnitt des Knochenkanals 3 befindet. Das andere Ende der Einfüllkanüle 2 ist mit einem (nicht dargestellten) Spender für das Verfüllmaterial 5, vorzugsweise Knochenzement, verbunden, welches in Richtung des Pfeils in die Kanüle eintritt.

Während des Verfüllens wird die Einfüllkanüle langsam zum Eingang des Knochenkanals bewegt, wobei das freie Ende 11 der Kanüle 2 aufgrund seiner radialen Elastizität auch dann eine ausreichende Abdichtung des Ringraumes zwischen Innenwandung des Knochenkanals 3 und der Außenwandung der Einfüllkanüle 2 gewährleistet, wenn der Innendurchmesser des Kanals 3 örtlich variiert.

Die Abdichtung gewährleistet eine Verpressung des Verfüllmaterials mit großer Dichte und unter Vermeidung von Lufteinschlüssen. Die größe Dichte des verfüllten Knochenzements und die daraus resultierende hohe Festigkeit der sich auf einem Verschlußelement 6 abstützenden Füllung 5, beruht darauf, daß sich der Kompressionsdruck nur über das Verfüllmaterial ausgleichen kann und damit im wesentlichen konstant bleibt. Da der Außendurchmesser des freien Endes der Einfüllkanüle durch seine erfindungsgemäße Ausbildung nur unwesentlich vergrößert wird, ist es nicht erforderlich, den Innendurchmesser des Knochenkanals 3 zusätzlich zu erweitern. Dadurch wird in vorteilhafter Weise vermieden, daß gesundes Knochenmaterial abgetragen werden muß, um den zu behandelnden Knochen entsprechend für die Implantation vorzubereiten.

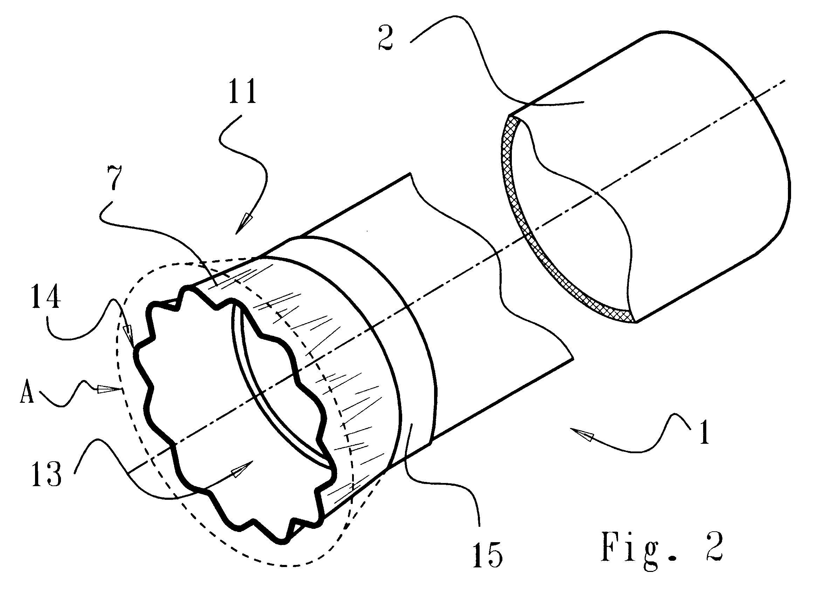

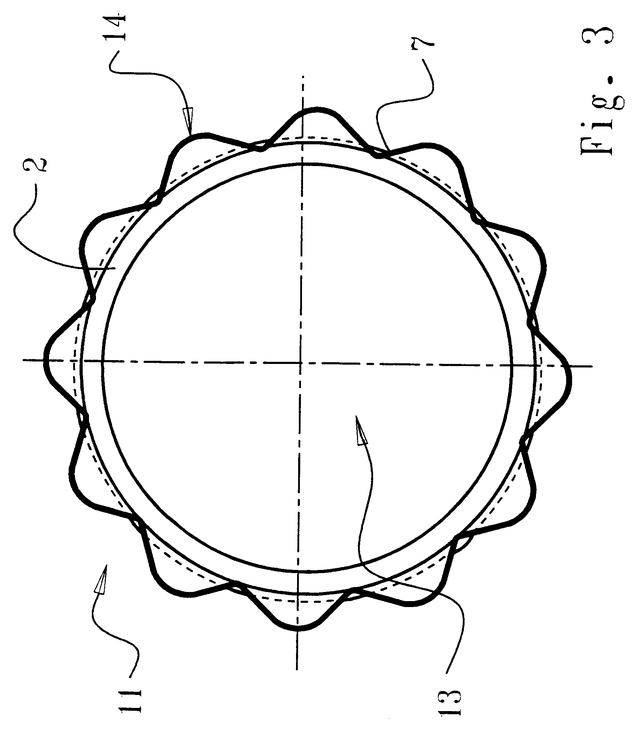

Die in Figur 2 perspektivisch und in Figur 3 in Draufsicht auf die Materialaustrittöffnung 13 dargestellte Einfüllkanüle 2 eines Ausführungsbeispiels der erfindungsgemäßen Einfüllvorrichtung für Knochenzement weist ein freies Ende 11 auf, an dem eine faltenrock-ähnliche Manschette 7 vorgesehen ist. Die Manschette 7 ist mit einem ihrer, zylindrischen ausgebildeten Enden 15 auf das freie Ende der Einfüllkanüle 2 aufgeschoben und dort durch Kleben befestigt. Die Manschette besteht aus einem elastischen Gummi und hat im Verhältnis zur Wandstärke der Einfüllkanüle 2 eine relativ geringe Dicke.

Während des Einfüllvorgangs wird die Manschette 7 radial gedehnt und die gewellte Begrenzungslinie 14 nimmt die Form einer Kreislinie an, wobei die Manschette 7 einen Kegelstumpf bildet (vergleiche Position A oder Figur 1)

Entsprechend einer nicht dargestellten Weiterbildung der Erfindung ist es günstig, die Manschette 7 direkt an das freie Ende 11 der Einfüllkanüle 2 anzuformen.

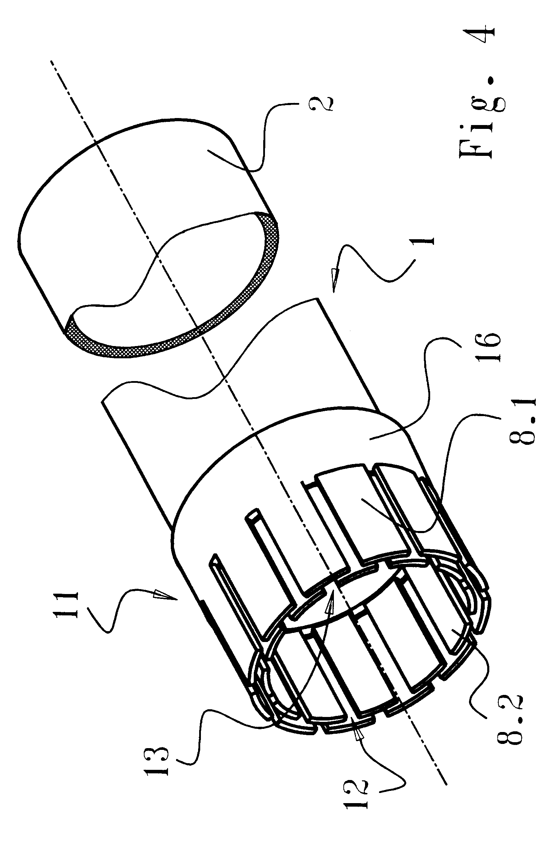

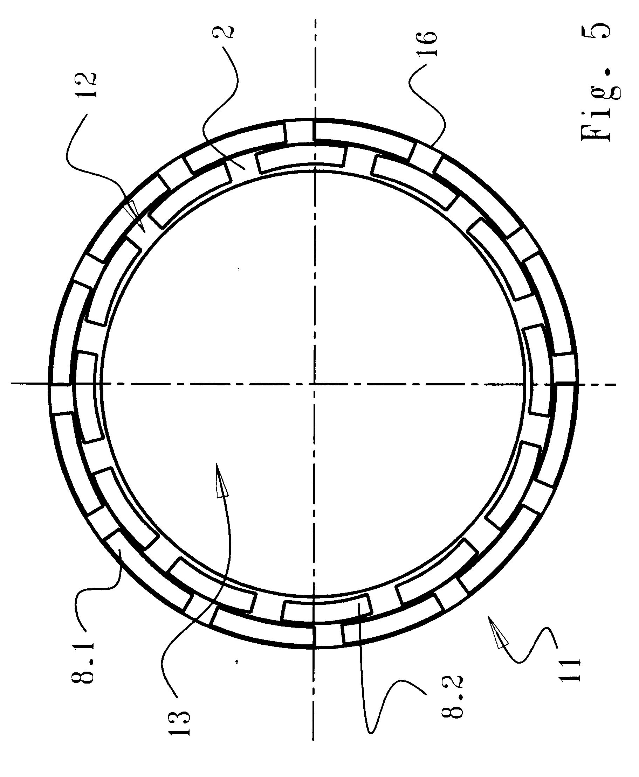

Die Figuren 4 und 5 zeigen in perspektivischer Darstellung und als Draufsicht auf die Materialeinbringöffnung 13 der Einfüllkanüle 2 eine andere vorteilhafte Ausführungsform der Erfindung. Das freie Ende 11 der Einfüllkanüle 2 besteht aus mehreren, gleichartig ausgebildeten Streifen 8.1, 8.2. Die Streifen sind gleichmäßig und symmetrisch am Umfang der Austrittsöffnung 13 der Einfüllkanüle 2in zwei Lagen verteilt angeordnet, wobei sich die einzelnen Streifen parallel zur Mittelachse der Einfüllkanüle 2 erstrecken und die Wandelemente eines doppelwandigen zylindrischen Hohlkörpers bilden. Die Streifen 8.2 der sind an die kreisringförmige Endfläche der Einfüllkanüle 2 angeformt, wobei zwischen den einzelnen Streifen jeweils ein Spalt 12 geringer Breite vorgesehen ist, der sich über die gesamte Länge der Streifen erstreckt.

Die Streifen 8.1 der zweiten Lage befinden sich dicht über den Streifen 8.2 der ersten Lage und sind derart angeordnet, daß sie die zwischen den Streifen 8.2 bestehenden Spalte 12 vollflächig überdecken. Die Breite der Streifen 8.1 ist so groß gewählt, daß die Bedeckung der Spalte 12 auch dann gewährleistet ist, wenn die beiden streifenlagen unter Einfluß des Verfüllmaterials radial nach außen ausgelenkt werden, um den Ringspalt zwischen der Innenwandung des Knochenkanals 3 und dem freien Ende 11 der Einfüllkanüle 2 gegen Durchtritt des Verfüllmaterials abzudichten. Die Streifen 8.1 sind an einem ringförmigen Überwurf 16 befestigt, der über den ungeschlitzten Teil des freien Ende 11 der Einfüllkanüle 2 geschoben und mit diesem durch Kleben oder ausreichende Haftreibung verbunden ist. Es besteht ebenso die (nicht dargestellte) Möglichkeit, auch die zweite Streifenlage direkt an das freie Ende 11 der Einfüllkanüle 2 anzuformen. Die Streifen 8.1 und 8.2 bestehen aus dem glöeichen Material wie die Einfüllkanüle 2, aus Gummi oder einem geeignet elastischen Kunststoff.

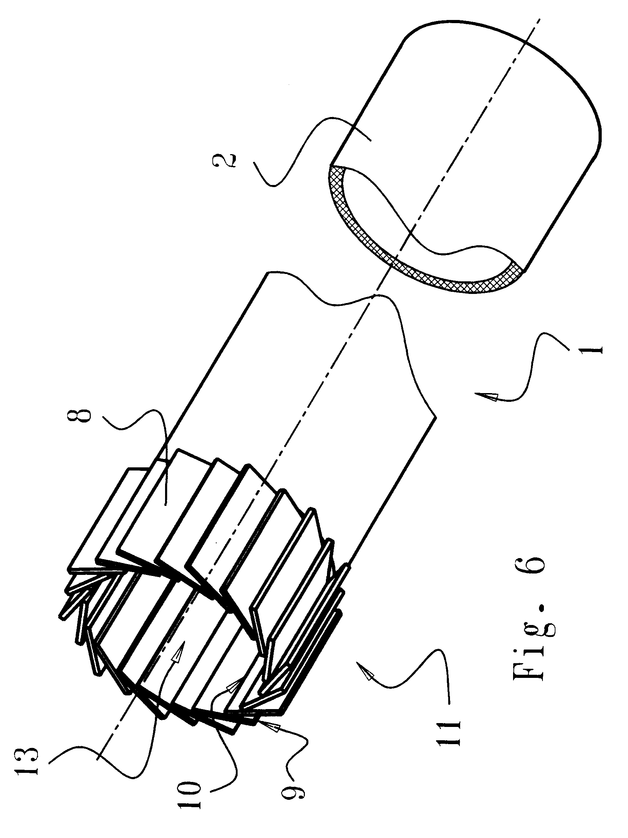

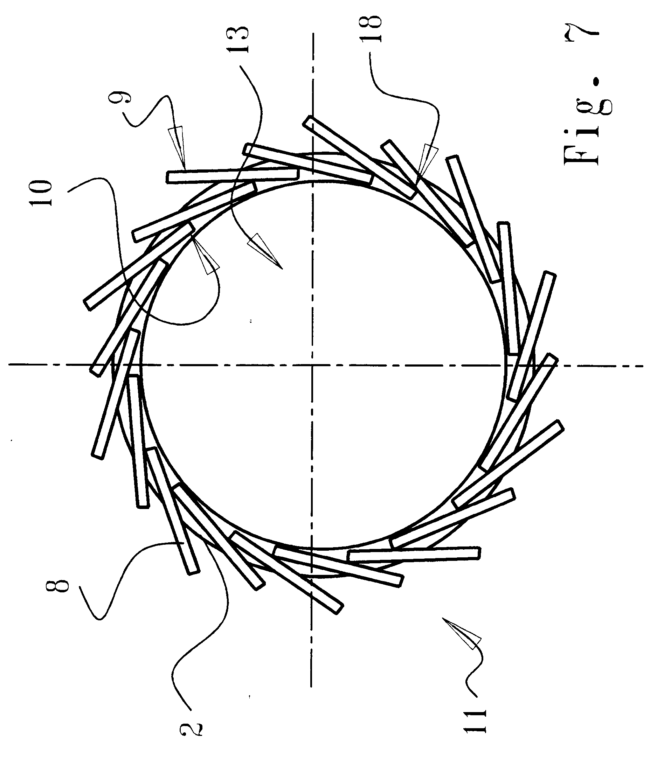

In den Figuren 6 und 7 ist eine weitere günstige Weiterbildung der Erfindung perspektivisch und als Draufsicht auf die Materialaustrittsöffnung 13 in schematisierter Form dargestellt. Die Streifen 8 sind im wesentlichen einlagig angeordnet und an die kreisringförmige Stirnfläche des freien Endes 11 der Einfüllkanüle 2 angeformt. Die Streifen 8 bilden Elemente der Wandung eines Hohlzylinders und sind unter Einwirkung des Verfüllmaterials mit ihrem freien Ende radial nach außen beweglich. Die Streifen 8 sind derart angeordnet, daß jeweils die in Umfangsrichtung hintere Längskante 10 eines Streifens die vordere Längskante 9 des nachfolgenden Streifens derart überragt, daß in Längserstreckung der Streifen eine durchgehende Dichtkante 18 gebildet wird, wobei sich die Streifen 8in ihren Querausdehnung im wesentlichen tangential bezüglich des Umfangs der Austrittsöffnung 13 der Einfüllkanüle 2 erstrecken.

Die Erfindung beschränkt sich in ihrer Ausgestaltung nicht auf die vorstehend angegebenen bevorzugten Ausführungsbeispiele. Vielmehr ist eine Anzahl von Varianten denkbar, welche von der dargestellten Lösung in den Grenzen der Ansprüche auch bei anders gearteten Ausführungen Gebrauch macht.

## Patentansprüche

1. Einfüllvorrichtung (1) für das Einbringen von Knochenzement (5) in den intramedullären Kanal (3) eines Knochens (4), mit einer Einfüllkanüle (2) und einem Leitmittel (7; 8; 8.1, 8.2) zur Führung des Knochenzements beim Austritt aus dem Ende der Kanüle, welches als flexible schlauchartige Manschette ausgebildet ist, welche an einem ihrer Enden (15) im Bereich der Austrittsöffnung (13) der Einfüllkanüle (2) dichtend befestigt ist und zu ihrem freien Ende (11) hin zur Anlage an den Innenseite des intramedullären Kanals durch den unter Druck austretenden Knochenzement in radialer Richtung expandierbar ist, **dadurch gekennzeichnet**, daß das Leitmittel eine zu seinem freien Ende hin zunehmende, durch Überlappung, gebildet durch eine lamellenartige Schlitzung des Leitmittels in Austrittsrichtung des Knochenzements, oder Elastizität gebildete Flächenreserve zur radialen Expansion aufweist, die trotz Aufspreizens des freien Endes durch den austretenden Knochenzement eine geschlossene Manschette bildet, welche ein Aufsteigen des Knochenzements (5) an der Kanüle (2) verhindert.

2. Einfüllvorrichtung nach Anspruch 1, **dadurch gekennzeichnet**, daß die Verformbarkeit des Leitmittels (7; 8; 8.1, 8.2) in Austrittsrichtung des Verfüllmaterials zunimmt.

3. Einfüllvorrichtung nach Anspruch 1, **dadurch gekennzeichnet**, daß die lamellenartige Schlitzung in Form von in Längsrichtung verlaufenden Streifenelementen (8; 8.1, 8.2) ausgestaltet ist.

4. Einfüllvorrichtung nach Anspruch 3, **dadurch gekennzeichnet**, daß die Streifenelemente zwei- oder mehrlagig ausgebildet sind, wobei die Streifenelemente (8.1) der oberen Lage jeweils die zwischen den Streifenelementen (8.2) der unteren Lage befindlichen Stoßkanten bzw. Spalten (12) überdecken.

5. Einfüllvorrichtung nach Anspruch 4, **dadurch gekennzeichnet**, daß sich die maximale Ausdehnung des Querschnitts der Streifenelemente (8; 8.1, 8.2) in im wesentlichen tangentialer Richtung der Einfüllkanüle (2) erstreckt.

6. Einfüllvorrichtung nach Anspruch 3, **dadurch gekennzeichnet**, daß die Streifenelemente (8) im wesentlichen einlagig angeordnet sind, wobei jeweils die in Umfangsrichtung hintere Längskante (10) eines Streifenelementes (8) die vordere Längskante (9) des nachfolgenden Streifenelementes mindestens in der Ruhestellung im Querschnitt sägezahnartig überragt.

7. Einfüllvorrichtung nach einem der vorangehenden Ansprüche, **dadurch gekennzeichnet**, daß das Leitmittel aus im wesentlichen demselben Material besteht wie die Einfüllkanüle (2) und sich insbesondere einstückig an diese anschließt.

8. Einfüllvorrichtung nach einem der vorangehenden Ansprüche, **dadurch gekennzeichnet**, daß das Leitmittel aus einem Gummimaterial oder aus einem elastischen Kunststoff besteht.

9. Einfüllvorrichtung nach einem der vorangehenden Ansprüche, **dadurch gekennzeichnet**, daß das Leitmittel (7) am freien Ende (11) der Einfüllkanüle (2) aufgeschoben und/oder durch Kleben befestigt ist.

## Claims

1. A filling device (1) for introducing bone cement (5) into the intramedullary canal (3) of a bone (4), having a filling cannula (2) and a guide means (7; 8; 8.1, 8.2) for guiding the bone cement on issuing from the end of the cannula, in the form of a flexible hose-like collar which at one of its ends (15) is sealingly fixed in the region of the outlet opening (13) of the filling cannula (2) and which is expandable in the radial direction towards its free end (11) to bear against the inside of the intramedullary canal by the bone cement which issues under pressure, characterised in that the guide means has a surface reserve for the purposes of radial expansion, which surface reserve increases towards its free end and is formed by overlapping, formed by plate-like slitting of the guide means in the direction of issue of the bone cement, or elasticity, and which in spite of spreading of the free end by the issuing bone cement forms a closed collar which prevents the bone cement (5) from rising up along the cannula (2).

2. A filling device according to claim 1 characterised in that the deformability of the guide means (7; 8; 8.1, 8.2) increases in the direction of issue of the filling material.

3. A filling device according to claim 1 characterised in that the plate-like slitting is in the form of strip elements (8; 8.1, 8.2) extending in the longitudinal direction.

4. A filling device according to claim 3 characterised in that the strip elements are of a configuration involving two or more layers, wherein the strip elements (8.1) of the upper layer respectively cover over the gaps or join edges between the strip elements (8.2) of the lower layer.

5. A filling device according to claim 4 characterised in that the maximum extent of the cross-section of the strip elements (8; 8.1, 8.2) extends in a substantially tangential direction of the filling cannula (2).

6. A filling device according to claim 3 characterised in that the strip elements (8) are arranged substantially in a single-layer configuration, wherein in each case the longitudinal edge (10) of a strip element (8), which is the trailing edge in the peripheral direction, projects sawtooth-like over the leading longitudinal edge (9) of the subsequent strip element in cross-section at least in the rest position.

7. A filling device according to one of the preceding claims characterised in that the guide means comprises substantially the same material as the filling cannula (2) and in particular integrally adjoins same.

8. A filling device according to one of the preceding claims characterised in that the guide means comprises a rubber material or an elastic plastic material.

9. A filling device according to one of the preceding claims characterised in that the guide means (7) is pushed on at the free end (11) of the filling cannula (2) and/or is fixed by adhesive.

## Revendications

1. Dispositif de remplissage (1) destiné à l'introduction de ciment osseux (5) dans le canal intramédullaire (3) d'un os (4), comportant une canule de remplissage (2) et un moyen de guidage (7 ; 8 ; 8.1, 8.2) destiné à guider le ciment osseux à sa sortie de l'extrémité de la canule, lequel est conçu en tant que manchon de type tube souple fixé de façon étanche à une de ses extrémités (15) dans la zone de l'ouverture de sortie (13) de la canule de remplissage (2) et expansible dans la direction radiale à son extrémité libre (11) pour appui contre la face interne du canal intramédullaire par le ciment osseux sortant sous pression, caractérisé en ce que le moyen de guidage présente une réserve de surface pour expansion radiale augmentant en direction de son extrémité libre, formée par recouvrement, constitué par un entaillage en lamelles du moyen de guidage dans la direction de sortie du ciment osseux, ou par élasticité, et qui, en dépit de l'écartement de l'extrémité libre par le ciment osseux sortant, forme un manchon fermé qui empêche une montée du ciment osseux (5) le long de la canule (2).

2. Dispositif de remplissage selon la revendication 1, caractérisé en ce que la déformabilité du moyen de guidage (7 ; 8 ; 8.1, 8.2) augmente dans la direction de sortie du matériau de remplissage.

3. Dispositif de remplissage selon la revendication 1, caractérisé en ce que l'entaillage en lamelles est conçu sous la forme d'éléments de type bande (8 ; 8.1, 8.2) s'étendant dans le sens longitudinal.

4. Dispositif de remplissage selon la revendication 3, caractérisé en ce que les éléments de type bande sont constitués en deux ou plusieurs couches, les éléments de type bande (8.1) de la couche supérieure recouvrant les rebords ou fentes (12) se trouvant entre les éléments de type bande (8.2) de la couche inférieure respective.

5. Dispositif de remplissage selon la revendication 4, caractérisé en ce que l'extension maximale de la section transversale des éléments de type bande (8 ; 8.1, 8.2) s'étend dans sensiblement la direction tangentielle de la canule de remplissage (2).

6. Dispositif de remplissage selon la revendication 3, caractérisé en ce que les éléments de type bande (8) sont disposés en sensiblement une couche, l'arête longitudinale (10) arrière dans la direction périphérique d'un élément de type bande (8) dépassant en dents de scie en section transversale de l'arête longitudinale avant (9) de l'élément de type bande suivant au moins dans la position de repos.

7. Dispositif de remplissage selon une des revendications précédentes, caractérisé en ce que le moyen de guidage est en sensiblement le même matériau que la canule de remplissage (2) et lui fait notamment suite de façon monobloc.

8. Dispositif de remplissage selon une des revendications précédentes, caractérisé en ce que le moyen de guidage est en un matériau caoutchouteux ou en une matière plastique élastique.

9. Dispositif de remplissage selon une des revendications précédentes, caractérisé en ce que le moyen de guidage (7) est enfilé sur l'extrémité libre (11) de la canule de remplissage (2) et/ou fixé par collage.
